(19) Europäisches Patentamt
European Patent Office
Office européen des brevets

(11) Veröffentlichungsnummer: **0 270 002 B1**

(12) **EUROPÄISCHE PATENTSCHRIFT**

(45) Veröffentlichungstag der Patentschrift: **15.04.92**

(51) Int. Cl.[5]: **C07C 49/713**, C07C 49/723, C07C 49/753, C07C 49/835, C07C 45/54, C07C 45/67, C07C 45/64, C07C 69/78, C07C 69/145, A01N 35/06

(21) Anmeldenummer: **87117470.2**

(22) Anmeldetag: **26.11.87**

(54) **Cyclohexenonderivate.**

(30) Priorität: **03.12.86 DE 3641234**

(43) Veröffentlichungstag der Anmeldung:
**08.06.88 Patentblatt 88/23**

(45) Bekanntmachung des Hinweises auf die Patenterteilung:
**15.04.92 Patentblatt 92/16**

(84) Benannte Vertragsstaaten:
**BE CH DE FR GB IT LI NL**

(56) Entgegenhaltungen:
**EP-A- 0 070 495**
**EP-A- 0 233 568**
**US-A- 4 175 135**

(73) Patentinhaber: **BASF Aktiengesellschaft**
**Carl-Bosch-Strasse 38**
**W-6700 Ludwigshafen(DE)**

(72) Erfinder: **Keil, Michael, Dr.**
**Fontanestrasse 4**
**W-6713 Freinsheim(DE)**
Erfinder: **Schirmer, Ulrich, Dr.**
**Berghalde 79**
**W-6900 Heidelberg(DE)**
Erfinder: **Kolassa, Dieter, Dr.**
**Moltkestrasse 8**
**W-6700 Ludwigshafen(DE)**
Erfinder: **Kast, Juergen, Dr.**
**Kastanienstrasse 24**
**W-6737 Boehl-Iggelheim(DE)**
Erfinder: **Rademacher, Wilhelm, Dr.**
**Austrasse 1**
**W-6703 Limburgerhof(DE)**
Erfinder: **Jung, Johann, Prof. Dr.**
**Hardenburgstrasse 19**
**W-6703 Limburgerhof(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 270 002 B1

## Beschreibung

Die Erfindung betrifft neue Cyclohexenonderivate I, Verfahren zu ihrer Herstellung und ihre Verwendung als Mittel, die das Wachstum von Pflanzen regulieren sowie ein Verfahren zur Regulierung des Pflanzenwachstums.

Aus EP-A-123 001 und EP-A-126 713 ist bekannt, daß bestimmte 2-Acyl-3-hydroxycyclohex-2-en-1-one das Pflanzenwachstum beeinflussen.

Es wurde nun gefunden, daß Cyclohexenonderivate der Formel I

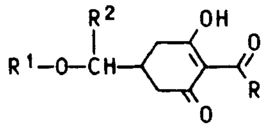

(I),

in der

R $C_1$-$C_6$-Alkyl, Cyclopropyl, Phenyl oder $C_2$-$C_6$-Alkoxyalkyl,

$R^1$ Wasserstoff, Tetrahydropyran-2-yl, Benzoyl oder gegebenenfalls halogensubst. $C_1$-$C_6$-Alkanoyl und

$R^2$ Wasserstoff oder $C_1$-$C_6$-Alkyl

bedeuten, oder pflanzenphysiologisch verträgliche Salze dieser Verbindungen vorteilhafte Eigenschaften als Wachstumsregulatoren und auch hinsichtlich der Verträglichkeit für Pflanzen aufweisen. Die Verwendung der Verbindungen als Wachstumsregulatoren unterliegt den Grundsätzen, wie sie für vergleichbar wirkende Verbindungen beschrieben sind, beispielsweise in einer der vorstehenden Druckschriften.

Im einzelnen haben die Substituenten in Formel I folgende Bedeutungen:

R
- unverzweigtes und verzweigtes $C_1$-$C_6$-Alkyl, bevorzugt $C_1$-$C_5$-Alkyl, wie Methyl, Ethyl, n-Propyl, n-Butyl und n-Pentyl,
- Cyclopropyl,
- Phenyl,
- $C_2$-$C_6$-Alkoxyalkyl, bevorzugt $C_2$-$C_4$-Alkoxyalkyl, wie Methoxymethyl, Ethoxymethyl, n-Propoxymethyl, iso-Propoxymethyl, 1-Methoxyethyl, 2-Methoxyethyl, 1-Ethoxyethyl, 2-Ethoxyethyl, 1-Methoxy-n-propyl, 2-Methoxy-n-propyl, 3-Methoxy-n-propyl, 1-Methoxy-isopropyl und 2-Methoxy-isopropyl, besonders bevorzugt 2-Methoxyethyl,

$R^1$
- Wasserstoff,
- unverzweigtes und verzweigtes $C_1$-$C_6$-Alkanoyl, bevorzugt unverzweigtes $C_1$-$C_5$-Alkanoyl, wie Formyl, Acetyl, n-Propionyl, n-Butyryl, Valeryl und Caproyl,
- durch Halogen substituiertes unverzweigtes oder verzweigtes $C_1$-$C_6$-Alkanoyl, bevorzugt durch Chlor ein- bis dreifach substituiertes $C_2$- und $C_3$-Alkanoyl, wie Chloracetyl, Dichloracetyl, Trichloracetyl, 2-Chlorpropionyl und 3-Chlorpropionyl,
- Benzoyl,
- Tetrahydropyran-2-yl,

$R_2$
- Wasserstoff,
- unverzweigtes oder verzweigtes $C_1$-$C_6$-Alkyl, bevorzugt unverzweigtes oder verzweigtes $C_1$-$C_3$-Alkyl, wie Methyl, Ethyl, n-Propyl und iso-Propyl.

Als Salze der Verbindungen der Formel I kommen pflanzenphysiologisch verträgliche Salze, beispielsweise von Alkalimetallen, insbesondere Kalium oder Natrium, Erdalkalimetallen, insbesondere Calcium, Magnesium oder Barium, ferner von Mangan, Kupfer, Zink oder Eisen sowie Ammonium-, Phosphonium-, Sulfonium- oder Sulfoxoniumsalze, beispielsweise Ammonium-, Tetraalkylammonium-, Benzyltrialkylammonium-, Trialkylsulfonium- oder Trialkylsulfoxoniumsalze in Betracht.

Die Cyclohexenonderivate der Formel I können durch Umsetzung einer entsprechenden Verbindung der Formel II

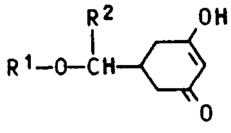

(II),

in üblicher Weise mit einem Säurechlorid R-COCl, in Gegenwart einer Base, wie Triethylamin, Pyridin, Natriumhydroxid, Kaliumcarbonat zum Enolester und anschließende Umlagerung, in Gegenwart einer sauren oder basischen Katalysator in einem inerten Lösungsmittel (z.B. Essigester, Toluol) bei Temperaturen zwischen 0 und 100 ° C hergestellt werden. Beispiele für saure Katalysatoren sind Aluminiumchlorid und Eisenchlorid; Beispiele für basische Katalysatoren sind vorzugsweise Imidazolderivate, z.B. Imidazol, oder Pyridinderivate, beispielsweise 4-N, N-Dimethylaminopyridin.

Die Alkohole, d.h. die Verbindungen, bei denen $R^1$ Wasserstoff ist, gewinnt man zweckmäßig aus den entsprechenden, zunächst mit einer Schutzgruppe versehenen Verbindungen. $R^1$, die Schutzgruppe kann z.B. tert. Butyl, Tetrahydropyran-2-yl oder Benzoyl sein, die man in üblicher Weise mit Hilfe einer Säure z.B. Salzsäure, Schwefelsäure, Phosphorsäure bzw. (bei Benzyl) hydrogenolytisch, z.B. mit Wasserstoff in Anwesenheit von Pd/C abspaltet. Auch die $NaBH_4$-Reduktion geeigneter Vorprodukte führt zum Ziel:

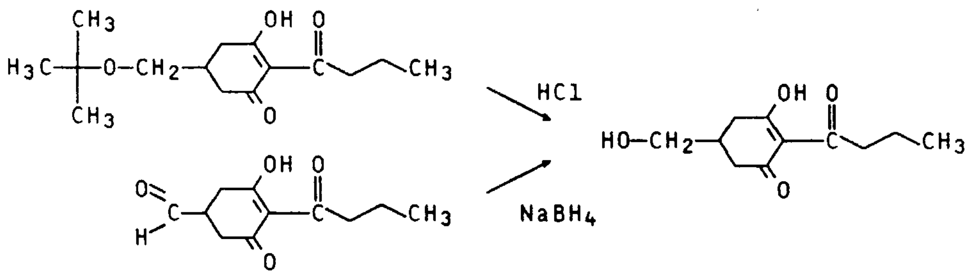


Die für die Umsetzung benötigten Vorprodukte lassen sich nach dem nachstehenden Schema gewinnen:

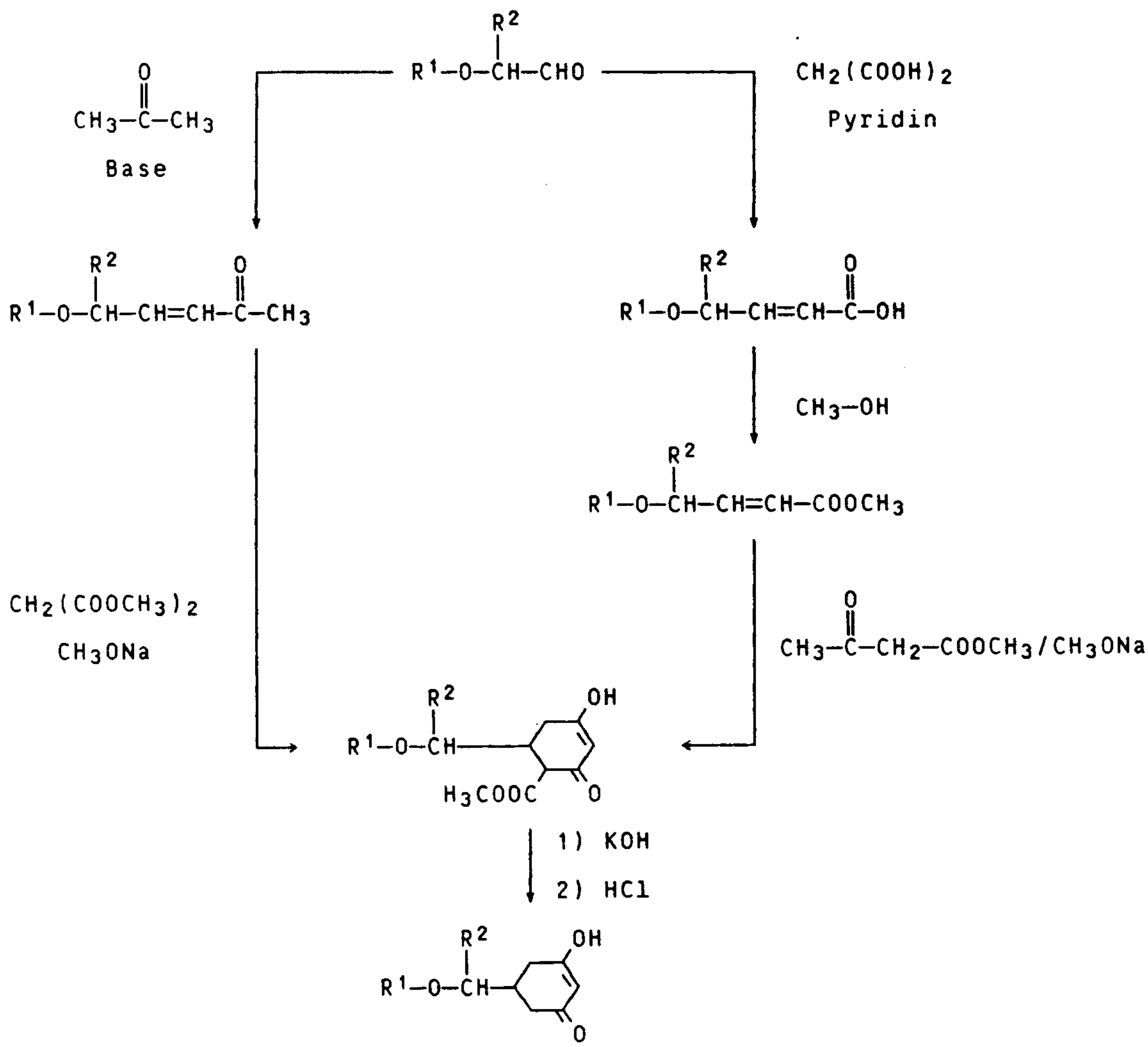


Herstellungsbeispiele

Beispiel 1

Zu 2, 0 g 2-Butyryl-5-formyl-3-hydroxy-cyclohex-2-en-1-on in 30 ml wäßrigem Tetrahydrofuran wird 0, 1 g Natriumborhydrid zugesetzt und 2 Stunden bei 25 ° C gerührt. Anschließend wird mit Wasser verdünnt, mehrfach mit Diethylether extrahiert, getrocknet und eingeengt. Man erhält 1, 5 g 2-Butyryl-3-hydroxy-5-hydroxymethylcyclohex-2-en-1-on (Verbindung Nr. 3).

Beispiel 2

44, 6 g 2-Butyryl-5-tert. - butoxymethyl-3-hydroxy-cyclohex-2-en-1-on werden in Ethanol gelöst und bei 0 bis 10 ° C mit 600 ml konz. Salzsäure versetzt. Nach 2 Stunden wird eingeengt, der Rückstand in Dichlormethan aufgenommen und mit Wasser ausgeschüttelt. Nach Einengen erhält man 26, 2 g 2-Butyryl-3-hydroxy-5-hydroxymethyl-cyclohex-2-en-1-on als hellgelbes Öl (Verbindung Nr. 3).

Die zur Charakterisierung und Struktursicherung geeigneten $^1$H-NMR-Spektren der erfindungsgemäßen Verbindungen werden in Deuterochloroform als Lösungsmittel mit Tetramethylsilan als internem Standard aufgenommen. Die $^1$H-chemischen Verschiebungen sind jeweils in (ppm) angegeben, wobei nur charakteristische Signale genannt sind. Für die Signalstruktur gelten folgende Abkürzungen:
s = Singulett, d = Dublett, t = Triplett, q = Quartett, m = Multiplett.

Tabelle

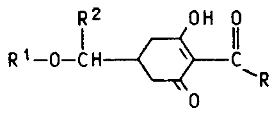

(I)

| Verbindung Nr. | R | $R^2$ | $R^1$ | δ [ppm] |
|---|---|---|---|---|
| 1 | $CH_3$ | H | H | 1,9-2,9 (m); 2,61 (s); 3,67 (m) |
| 2 | $C_2H_5$ | H | H | 1,12 (t); 3,04 (q), 3,62 (t) |
| 3 | $n-C_3H_7$ | H | H | 0,98 (t); 3,01 (t); 3,64 (m) |
| 4 | $n-C_4H_9$ | H | H | |
| 5 | $n-C_5H_{11}$ | H | H | |
| 6 | Cyclopropyl | H | H | 1,12 (m); 1,28 (m); 3,58 (m) |
| 7 | Phenyl | H | H | |
| 8 | 2-Methoxyethyl | H | H | |
| 9 | $CH_3$ | $CH_3$ | H | |
| 10 | $C_2H_5$ | $CH_3$ | H | |
| 11 | $n-C_3H_7$ | $CH_3$ | H | 0,98 (t); 1,25 (d); 3,76 (m) |
| 12 | $n-C_4H_9$ | $CH_3$ | H | |
| 13 | Cyclopropyl | $CH_3$ | H | |
| 14 | Phenyl | $CH_3$ | H | |
| 15 | 2-Methoxyethyl | $CH_3$ | H | |
| 16 | $C_2H_5$ | $C_2H_5$ | H | |
| 17 | $n-C_3H_7$ | $C_2H_5$ | H | 1,04 (m); 2,99 (t); 3,44 (m) |
| 18 | $C_2H_5$ | $i-C_3H_7$ | H | |
| 19 | $n-C_3H_7$ | $i-C_3H_7$ | H | |
| 20 | $C_2H_5$ | H | $CH_3CO$ | 1,13 (t); 2,11 (s); 3,04 (q) |
| 21 | $n-C_3H_7$ | H | $CH_3CO$ | 0,98 (t); 2,08 (s); 4,04 (m) |
| 22 | $C_2H_5$ | H | $C_2H_5CO$ | 1,14 (m); 3,07 (q); 4,16 (q) |
| 23 | $n-C_3H_7$ | H | $C_2H_5CO$ | 0,99 (t); 1,15 (t); 3,02 (t) |
| 24 | $C_2H_5$ | H | $n-C_3H_7CO$ | |
| 25 | $n-C_3H_7$ | H | $n-C_3H_7CO$ | 0,98 (m); 3,02 (t); 4,05 (m) |
| 26 | $C_2H_5$ | H | $n-C_5H_{11}CO$ | |
| 27 | $n-C_3H_7$ | H | $n-C_5H_{11}CO$ | 0,91 (t); 0,98 (t); 4,07 (d) |
| 28 | $C_2H_5$ | H | HCO | |
| 29 | $n-C_3H_7$ | H | HCO | |
| 30 | $n-C_4H_9$ | H | HCO | |
| 31 | $C_2H_5$ | H | $Cl-CH_2CO$ | |
| 32 | $n-C_3H_7$ | H | $Cl-CH_2CO$ | 0,98 (t); 4,10 (s); 4,18 (m) |
| 33 | $C_2H_5$ | H | $Cl-CH_2CH_2CO$ | 1,13 (t); 3,08 (q); 3,76 (t) |
| 34 | $n-C_3H_7$ | H | $Cl-CH_2CH_2CO$ | 0,98 (t); 3,01 (t); 3,77 (t) |

Tabelle (Forts.)

| Verbindung Nr. | R | $R^2$ | $R^1$ | δ [ppm] |
|---|---|---|---|---|
| 35 | $C_2H_5$ | H | $Cl_3CCO$ | |
| 36 | n-$C_3H_7$ | H | $Cl_3CCO$ | 0,98 (t); 3,03 (t); 4,32 (m) |
| 37 | $C_2H_5$ | H | $C_6H_5CO$ | |
| 38 | n-$C_3H_7$ | H | $C_6H_5CO$ | |
| 39 | $C_2H_5$ | H | Tetrahydropyran-2-yl | |
| 40 | n-$C_3H_7$ | H | Tetrahydropyran-2-yl | |
| 41 | $CH_3$ | $CH_3$ | $CH_3CO$ | |
| 42 | $C_2H_5$ | $CH_3$ | $CH_3CO$ | |
| 43 | n-$C_3H_7$ | $CH_3$ | $CH_3CO$ | 0,98 (t); 1,38 (d); 2,09 (s) |
| 44 | n-$C_4H_9$ | $CH_3$ | $CH_3CO$ | |
| 45 | n-$C_5H_{11}$ | $CH_3$ | $CH_3CO$ | |
| 46 | Cyclopropyl | $CH_3$ | $CH_3CO$ | |
| 47 | Phenyl | $CH_3$ | $CH_3CO$ | |
| 48 | 2-Methoxyethyl | $CH_3$ | $CH_3CO$ | |
| 49 | $C_2H_5$ | i-$C_3H_7$ | $CH_3CO$ | |
| 50 | n-$C_3H_7$ | i-$C_3H_7$ | $CH_3CO$ | |
| 51 | $CH_3$ | H | $CH_3CO$ | 2,09 (s); 2,61 (s); 4,03 (m) |
| 52 | n-$C_4H_9$ | H | $CH_3CO$ | |
| 53 | $CH_3$ Natriumsalz | H | H | |
| 54 | $C_2H_5$ Natriumsalz | H | H | |
| 55 | $C_2H_5$ Kaliumsalz | H | H | |
| 56 | $C_2H_5$ Tetrabutylammoniumsalz | H | H | |
| 57 | n-$C_3H_7$ Natriumsalz | H | H | |
| 58 | n-$C_3H_7$ Kaliumsalz | H | H | |
| 59 | n-$C_3H_7$ Tetrabutylammoniumsalz | H | H | |
| 60 | Cyclopropyl Natriumsalz | H | H | |
| 61 | n-$C_3H_7$ Natriumsalz | $C_2H_5$ | H | 0,83 (t); 0,86 (t); 3,08 (m) |
| 62 | n-$C_3H_7$ | $C_2H_5$ | $CH_3CO$ | 2,19 (s), 2,99 (t); 4,83 (m) |
| 63 | n-$C_3H_7$ Kaliumsalz | $CH_3$ | H | 0,80 (t), 0,98 (d); 3,35 (m) |
| 64 | n-$C_3H_7$ Natriumsalz | $CH_3$ | H | 0,82 (t); 1,02 (d); 3,36 (m) |
| 65 | Cyclopropyl | H | $CH_3CO$ | 1,14 (m); 1,31 (m); 2,09 (s) |

Die Cyclohexenonderivate der Formel I können praktisch alle Entwicklungsstadien einer Pflanze verschiedenartig beeinflussen und werden deshalb als Wachstumsregulatoren eingesetzt. Die Wirkungsvielfalt der Pflanzenwachstumsregulatoren hängt ab vor allem

a) von der Pflanzenart und - sorte,

b) von dem Zeitpunkt der Applikation, bezogen auf das Entwicklungsstadium der Pflanze und von der

Jahreszeit,

c) von dem Applikationsort und - verfahren (Samenbeize, Bodenbehandlung oder Blattapplikation)

d) von klimatischen Faktoren, z.B. Temperatur, Niederschlagsmenge, außerdem auch Tageslänge und Lichtintensität

e) von der Bodenbeschaffenheit (einschließlich Düngung),

f) von der Formulierung bzw. Anwendungsform des Wirkstoffs und schließlich

g) von den angewendeten Konzentrationen der aktiven Substanz.

Aus der Reihe der verschiedenartigen Anwendungsmöglichkeiten der erfindungsgemäßen Pflanzenwachstumsregulatoren im Pflanzenanbau, in der Landwirtschaft und im Gartenbau, werden einige nachstehend erwähnt.

A. Mit den erfindungsgemäß verwendbaren Verbindungen läßt sich das vegetative Wachstum der Pflanzen stark hemmen, was sich insbesondere in einer Reduzierung des Längenwachstums äußert. Die behandelten Pflanzen weisen demgemäß einen gedrungenen Wuchs auf; außerdem ist eine dunklere Blattfärbung zu beobachten.

Als vorteilhaft für die Praxis erweist sich z.B. die Verringerung des Grasbewuchses an Straßenrändern, Decken, Kanalböschungen und auf Rasenflächen wie Park-, Sport- und Obstanlagen, Zierrasen und Flugplätzen, so daß der Arbeits- und kostenaufwendige Rasenschnitt reduziert werden kann.

Von wirtschaftlichem Interesse ist auch die Erhöhung der Standfestigkeit von lageranfälligen Kulturen wie Getreide, Mais, Sonnenblumen und Soja. Die dabei verursachte Halmverkürzung und Halmverstärkung verringern oder beseitigen die Gefahr des "Lagerns" (des Umknickens) von Pflanzen unter ungünstigen Witterungsbedingungen vor der Ernte.

Wichtig ist auch die Anwendung von Wachstumsregulatoren zur Hemmung des Längenwachstums und zur zeitlichen Veränderung des Reifeverlaufs bei Baumwolle. Damit wird ein vollständig mechanisiertes Beernten dieser wichtigen Kulturpflanzen ermöglicht.

Durch Anwendung von Wachstumsregulatoren kann auch die seitliche Verzweigung der Pflanzen vermehrt oder gehemmt werden. Daran besteht Interesse, wenn z.B. bei Tabakpflanzen die Ausbildung von Seitentrieben (Geiztrieben) zugunsten des Blattwachstums gehemmt werden soll.

Mit Wachstumsregulatoren läßt sich beispielsweise bei Winterraps auch die Frostresistenz erheblich erhöhen. Dabei werden einerseits das Längenwachstum und die Entwicklung einer zu üppigen (und dadurch besonders frostanfälligen) Blatt- bzw. Pflanzenmasse gehemmt. Andererseits werden die jungen Rapspflanzen nach der Aussaat und vor dem Einsetzen der Winterfröste trotz günstiger Wachstumsbedingungen im vegetativen Entwicklungsstadium zurückgehalten. Dadurch wird auch die Frostgefährung solcher Pflanzen beseitigt, die zum vorzeitigen Abbau der Blühhemmung und zum Übergang in die generativen Phase neigen. Auch bei anderen Kulturen, z.B. Wintergetreide ist es vorteilhaft, wenn die Bestände durch Behandlung mit erfindungsgemäßen Verbindungen im Herbst zwar gut bestockt werden, aber nicht zu üppig in den Winter hineingehen. Dadurch kann der erhöhten Frostempfindlichkeit und - wegen der relativ geringen Blatt- bzw. Pflanzenmasse - dem Befall mit verschiedenen Krankheiten (z.B. Pilzkrankheit) vorgebeugt werden. Die Hemmung des vegetativen Wachstums ermöglicht außerdem bei vielen Kulturpflanzen eine dichtere Bepflanzung des Bodens, so daß ein Mehrertrag, bezogen auf die Bodenfläche, erzielt werden kann.

B. Mit den neuen Mitteln lassen sich Mehereträge sowohl an Pflanzenteilen als auch an Pflanzeninhaltsstoffen erzielen. So ist es beispielsweise möglich, das Wachstum größerer Mengen an Knospen, Blüten, Blättern, Früchten, Samenkörnern, Wurzeln und Knollen zu induzieren, den Gehalt an Zucker in Zuckerrüben, Zuckerrohr sowie Zitrusfrüchten zu erhöhen, den Proteingehalt in Getreide oder Soja zu steigern oder Gummibäume zum vermehrten Latexfluß zu stimulieren.

Dabei können die Cyclohexenonderivate der Formel I Ertragssteigerungen durch Eingriffe in den pflanzlichen Stoffwechsel bzw. durch Förderung oder Hemmung des vegetativen und/oder des generativen Wachstums verursachen.

C. Mit Pflanzenwachstumsregulatoren lassen sich schließlich sowohl eine Verkürzung bzw. Verlängerung der Entwicklungsstadien als auch eine Beschleunigung bzw. Verzögerung der Reife der geernteten Pflanzenteile vor oder nach der Ernte erreichen.

Von wirtschaftlichem Interesse ist beispielsweise die Ernteerleichterung, die durch das zeitlich konzentrierte Abfallen oder Vermindern der Haftfestigkeit am Baum bei Zitrusfrüchten, Oliven oder bei anderen Arten und Sorten von Kern-, Stein- und Schalenobst ermöglicht wird. Derselbe Mechanismus, das heißt die Förderung der Ausbildung von Trenngewebe zwischen Frucht - bzw. Blatt- und Sproßteil der Pflanze ist auch für ein gut kontrollierbares Entblättern von Nutzpflanzen wesentlich.

D. Mit Wachstumsregulatoren kann weiterhin der Wasserverbrauch von Pflanzen reduziert werden. Dies ist besonders wichtig für landwirtschaftliche Nutzflächen, die unter einem hohen Kostenaufwand künstlich

bewässert werden müssen, z.B. in ariden oder semiariden Gebieten. Durch den Einsatz der erfindungsgemäßen Substanzen läßt sich die Intensität der Bewässerung reduzieren und damit eine kostengünstigere Bewirtschaftung durchführen. Unter dem Einfluß von Wachstumsregulatoren kommt es zu einer besseren Ausnutzung des vorhandenen Wassers, weil u.a.

- die Öffnungsweite der Stomata reduziert wird
- eine dickere Epidermis und Cuticula ausgebildet werden
- die Durchwurzelung des Bodens verbessert wird das Mikroklima im Pflanzenbestand durch einen kompakteren Wuchs günstig beeinflußt wird.

Die erfindungsgemäß zu verwendenden Wirkstoffe können den Kulturpflanzen sowohl vom Samen her (als Saatgutbeizmittel) als auch über den Boden, d.h. durch die Wurzel sowie durch Spritzung über das Blatt zugeführt werden.

Infolge der hohen Pflanzenverträglichkeit kann die Aufwandmenge stark variiert werden.

Bei der Saatgutbehandlung werden im allgemeinen Wirkstoffmengen von 0,001 bis 50 g je Kilogramm Saatgut, vorzugsweise 0,01 bis 10 g, benötigt.

Für die Blatt- und Bodenbehandlung sind im allgemeinen Gaben von 0,01 bis 10 kg/ha bevorzugt 0,05 bis 3 kg/ha als ausreichend zu betrachten.

Die erfindungsgemäßen Mittel können in Form üblicher Formulierungen angewendet werden wie Lösungen, Emulsionen, Suspensionen, Stäube, Pulver, Pasten und Granulate. Die Anwendungsformen richten sich ganz nach den Verwendungszwecken; sie sollen in jedem Fall eine feine und gleichmäßige Verteilung der wirksamen Substanz gewährleisten. Die Formulierungen werden in bekannter Weise hergestellt, z.B. durch Verstrecken des Wirkstoffs mit Lösungsmitteln und/oder Trägerstoffen, gegebenenfalls unter Verwendung von Emulgiermitteln und Dispergiermitteln, wobei im Falle der Benutzung von Wasser als Verdünnungsmittel auch andere organische Lösungsmittel als Hilfslösungsmittel verwendet werden können. Als Hilfsstoffe kommen dafür im wesentlichen in Frage: Lösungsmittel wie Aromaten (z.B. Xylol, Benzol), chlorierte Aromaten (z.B. Chlorbenzole), Paraffine (z.B. Erdölfraktionen), Alkohole (z.B. Methanol, Butanol), Amine (z.B. Ethanolamin), N, N-Dimethylformamid und Wasser; feste Trägerstoffe wie natürliche Gesteinsmehle (z.B. Kaoline, Tonerden, Talkum, Kreide) und synthetische Gesteinsmehle (z.B. hochdisperse Kieselsäure, Silikate); Emulgiermittel oder sonstige oberflächenaktive Mittel, wie nichtionogene und anionische Emulgatoren (z.B. Polyoxyethylen-Fettalkohol-Ether, Alkylsulfonate) und Dispergiermittel wie Lignin, Sulfitablaugen und Methylcellulose. Bevorzugt ist die Anwendung der erfindungsgemäßen Verbindungen in wäßriger Lösung gegebenenfalls unter Zusatz von mit Wasser mischbaren organischen Lösungsmitteln wie Methanol oder anderen niederen Alkoholen, Aceton, N, N-Dimethylformamid oder N-Methylpyrrolidon. Die Formulierungen enthalten im allgemeinen zwischen 0, 1 und 95 Gew. % Wirkstoff, vorzugsweise zwischen 0, 5 und 90 Gew. %.

Die Formulierungen bzw. die daraus hergestellten gebrauchsfertigen Zubereitungen, wie Lösungen, Emulsionen, Suspensionen, Pulver, Stäube, Pasten oder Granulate werden in bekannter Weise angewendet, beispielsweise im Vorauflaufverfahren, im Nachauflaufverfahren oder als Beizmittel.

Beispiele für Formulierungen sind:

I 20 Gewichtsteile der Verbindung des Beispiel 3 werden in 3 Gewichtsteilen des Natriumsalzes der Diisobutylnaphthalin-sulfonsäure, 17 Gewichtsteilen des Natriumsalzes einer Ligninsulfonsäure aus einer Sulfitablauge und 60 Gewichtsteilen pulverförmigem Kieselsäuregel gut vermischt und in einer Hammermühle vermahlen. Durch feines Verteilen der Mischung in 20 000 Gewichtsteilen Wasser erhält man eine Spritzbrühe, die 0, 1 Gew. % des Wirkstoffs enthält.

II 3 Gewichtsteile der Verbindung des Beispiels 3 werden mit 97 Gewichtsteilen feinteiligem Kaolin innig vermischt. Man erhält auf diese Weise ein Stäubemittel, das 3 Gew. % des Wirkstoffs enthält.

III 30 Gewichtsteile der Verbindung des Beispiels 3 werden in einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

IV 40 Gewichtsteile der Verbindung des Beispiels 3 werden mit 10 Teilen Natriumsalz eines Phenolsulfonsäure-harnstoff-formaldehyd-Kondensats, 2 Teilen Kieselgel und 48 Teilen Wasser innig vermischt. Man erhält eine stabile wäßrige Dispersion. Durch Verdünnen mit 100 000 Gewichtsteilen Wasser erhält man eine wäßrige Dispersion, die 0, 04 Gew. % Wirkstoff enthält.

V 20 Teile der Verbindung des Beispiels 3 werden mit 2 Teilen Calciumsalz der Dodecylbenzolsulfonsäure, 8 Teilen Fettalkoholpolyglykolether, 2 Teilen Natiumsalz eines Phenolsufonsäure-harnstoff-formaldehyd-Kondensats und 68 Teilen eines paraffinischen Mineralöls innig vermischt. Man erhält eine stabile ölige Dispersion.

VI Man vermischt 90 Gewichtsteile der Verbindung des Beispiels 3 mit 10 Gewichtsteilen N-Methyl-α-pyrrolidon und erhält eine Lösung, die zur Anwendung in Form kleinster Tropfen geeignet ist.

VII 20 Gewichtsteile der Verbindung des Beispiels 3 werden in einer Mischung gelöst, die aus 80 Gewichtsteilen Xylol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Ricinusöl besteht. Durch Ausgießen und feines Verteilen der Lösung in 100 000 Gewichtsteilen Wasser erhält man eine wäßrige Dispersion, die 0, 02 Gew. % des Wirkstoffs enthält.

VIII 20 Gewichtsteile der Verbindung des Beispiels 3 werden in einer Mischung gelöst, die aus 40 Gewichtsteilen Cyclohexanon, 30 Gewichtsteilen Isobutanol, 20 Gewichtsteilen des Anlagerungsproduktes von 7 Mol Ethylenoxid an 1 Mol Isooctylphenol und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0, 02 Gew. % des Wirkstoffs enthält.

IX 20 Gewichtsteile der Verbindung des Beispiels 3 werden in einer Mischung gelöst, die aus 25 Gewichtsteilen Cyclohexanol, 65 Gewichtsteilen einer Mineralölfraktion vom Siedepunkt 210 bis 280 °C und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0, 02 Gew. % des Wirkstoffs enthält.

Die erfindungsgemäßen Mittel können in diesen Anwendungsformen auch zusammen mit anderen Wirkstoffen vorliegen, wie z.B. Herbiziden, Insektiziden, Wachstumsregulatoren und Fungiziden, oder auch mit Düngemitteln vermischt und ausgebracht werden. Beim Vermischen mit Wachstumsregulatormischungen treten auch synergistische Effekte auf, d.h. die Wirksamkeit des Kombinationsproduktes ist größer als die addierten Wirksamkeiten der Einzelkomponenten.

Fungizide, die mit den erfindungsgemäßen Verbindungen kombiniert werden können, sind beispielsweise:

Schwefel,
Dithiocarbamate und deren Derivate, wie
Ferridimethyldithiocarbamat,
Zinkdimethyldithiocarbamat,
Zinkethylenbisdithiocarbamat,
Manganethylenbisdithiocarbamat,
Mangen-Zink-ethylendiamin-bis-dithiocarbamat,
Tetramethylthiuramdisulfide,
Ammoniak-Komplex von Zink- (N, N-ethylen-bis-dithiocarbamat),
Ammoniak-Komplex von Zink- (N, N' - propylen-bis-dithiocarbamat),
Zink- (N, N' - propylen-bis-dithiocarbamat),
N, N' - Polypropylen-bis- (thiocarbamoyl) -disulfid;
Nitroderivate, wie
Dinitro- (1-methylheptyl) -phenylcrotonat,
2-sec-Butyl-4, 6-dinitrophenyl-3, 3-dimethylacrylat,
2-sec-Butyl-4, 6-dinitrophenyl-isopropylcarbonat;
5-Nitro-isophthalsäure-di-isopropylester
heterocyclische Substanzen, wie
2-Heptadecyl-2-imidazolin-acetat,
2, 4-Dichlor-6- (o-chloranilino)-s-triazin,
0, 0-Diethyl-phthalimidophosphonothioat,
5-Amino-1- [bis- (dimethylamino) - phosphinyl] -3-phenyl-1, 2, 4-triazol,
2, 3-Dicyano-1, 4-dithioanthrachinon,
2-Thio-1, 3-dithio- (4, 5-b) - chinoxalin,
1- (Butylcarbamoyl)-2-benzimidazol-carbaminsäuremethylester,
2-Methoxycarbonylamino-benzimidazol,
2- (Furyl- (2)) - benzimidazol,
2- (Thiazolyl- (4)) - benzimidazol,
N- (1, 1, 2, 2-Tetrachlorethylthio) - tetrahydrophthalimid,
N- Trichlormethylthio-tetrahydrophthalimid,
N- Trichlormethylthio-phthalimid,
N-Dichlorfluormethylthio-N' ,N' - dimethyl-N-phenyl-schwefelsäurediamid,
5-Ethoxy-3-trichlormethyl- 1, 2, 3-thiadiazol,
2-Rhodanmethylthiobenzthiazol,

1, 4-Dichlor-2, 5-dimethoxybenzol,
4 - (2-Chlorphenylhydroazano)-3-methyl-5-isoxazolon,
Pyridin-2-thio-1-oxid,
8-Hydroxychinolin bzw. dessen Kupfersalz,
2, 3-Dihydro-5-carboxanilido-6-methyl-1, 4-oxathiin,
2, 3-Dihydro-5-carboxanilido-6-methyl-1, 4-oxathiin-4, 4-dioxid,
2-Methyl-5, 6-dihydro-4H-pyran-3-carbonsäure-anilin,
2-Methyl-furan-3-carbonsäureanilid,
2, 5-Dimethyl-furan-3-carbonsäureanilid,
2, 4, 5-Trimethyl-furan-3-carbonsäureanilid,
2, 5-Dimethyl-furan-3-carbonsäurecyclohexylamid,
N-Cyclohexyl-N-methoxy-2, 5-dimethyl-furan-3-carbonsäureamid,
2-Methyl-benzoesäure-anilid,
2-Iod-benzoesäure-anilid,
N-Formyl-N-morpholin-2, 2, 2-trichlorethylacetal,
Piperazin-1, 4-diylbis- (1- (2, 2, 2-trichlor-ethyl) - formamid,
1- (3, 4-Dichloranilino) - 1 -formylamino- 2, 2, 2-trichlorethan,
2, 6-Dimethyl-N-tridecyl-morpholin bzw. dessen Salze,
2, 6-Dimethyl-N-cyclodedecyl-morpholin bzw. dessen Salze,
N-[3- (p-tert. -Butylphenyl) -2-methylpropyl]-cis-2, 6-dimethylmorpholin,
N-[3- (p-tert. -Butylphenyl) -2-methylpropyl]-piperidin,
1-[2- (2, 4-Dichlorphenyl) - 4-ethyl-1, 3-dioxolan-2-yl-ethyl] - 1H-1, 2, 4-triazol
1-[2- (2, 4-Dichlorphenyl) -4-n-propyl-1, 3-dioxolan-2-yl-ethyl] -1H-1, 2, 4-triazol,
N- (n-Propyl) -N- (2, 4, 6-trichlorphenoxyethyl) -N' -imidazol-yl-harnstoff,
1- (4-Chlorphenoxy) -3, 3-dimethyl-1- (1H-1, 2, 4-triazol-1-yl) -2-butanon,
1 -(4-Chlorphenoxy) -3, 3-dimethyl-1- (1H-1, 2, 4-triazol-1-yl) -2-butanol,
α- (2-Chlorphenyl)-α- (4-chlorphenyl) -5-pyrimidin-methanol,
5-Butyl-2-dimethylamino-4-hydroxy-6-methyl-pyrimidin,
Bis - (p-chlorphenyl) -3-pyridinmethanol,
1, 2-Bis- (3-ethoxycarbonyl-2-thioureido) -benzol,
1, 2-Bis- (3-methoxycarbonyl-2-thioureido) -benzol,
sowie verschiedene Fungizide, wie
Dodecylguanidinacetat,
3-[3- (3, 5-Dimethyl-2-oxycyclohexyl) -2-hydroxyethyl] - glutarimid,
Hexachlorbenzol,
DL-Methyl-N- (2, 6-dimethyl-phenyl)-N-furoyl (2)-alaninat,
DL-N- (2, 6-Dimethyl-phenyl) -N- (2′ -methoxyacetyl) -alanin-methylester,
N- (2, 6-Dimethylphenyl) -N-chloracetyl-D, L-2-aminobutyrolacton,
DL-N- (2, 6-Dimethylphenyl) -N- (phenylacetyl) -alaninmethylester,
5-Methyl-5-vinyl-3- (3, 5-dichlorphenyl) -2, 4-dioxo- 1, 3-oxazolidin,
3-[3, 5-Dichlorphenyl (-5-methyl-5-methoxymethyl] -1, 3-oxazolidin-2, 4-dion,
3-[3, 5-Dichlorphenyl) - 1 -isopropylcarbamoylhydantoin,
N-(3, 5-Dichlorphenyl) - 1, 2-dimethylcyclopropan-1, 2-dicarbonsäureimid,
2-Cyano-[N-(ethylaminocarbonyl) -2-methoximino] -acetamid, 1-[2- (2, 4-Dichlorphenyl) -pentyl]-1H-1, 2, 4-triazol,
2, 4-Difluor-α- (1H- 1, 2, 4-triazolyl-1-methyl)-benzhydrylalkohol.

Anwendungsbeispiele

Zur Bestimmung der wachstumsregulierenden Eigenschaft der Prüfsubstanzen wurden Testpflanzen auf ausreichend mit Nährstoffen versorgtem Kultursubstrat in Kunststoffgefäßen von ca. 12, 5 cm Durchmesser und 500 ml Volumen angezogen.

Bei der Vorauflauf-Bodenbehandlung wurden die zu prüfenden Substanzen in wäßriger Aufbereitung auf das Saatbett gegossen; bei der Nachauflauf-Blattbehandlung wurden die zu prüfenden Substanzen in wäßriger Aufbereitung auf die Pflanzen gesprüht.

Die beobachtete wachstumsregulierende Wirkung wurde bei Versuchsende durch Wuchshöhenmessung belegt. Die so gewonnenen Meßwerte wurden zur Wuchshöhe der unbehandelten Pflanzen in Relation gesetzt.

Als Vergleichssubstanz diente Chlormequatchlorid (CCC). Gleichlaufend zur Reduzierung des Längenwachstums steigt die Farbintensität der Blätter an. Der erhöhte Chlorophyllgehalt läßt eine ebenfalls erhöhte Photosynthese und damit eine erhöhte Ertragsbildung erwarten.

Tabelle 1a

| Sommergerste, Sorte "Aramir", Vorauflaufbehandlung (Bodenbehandlung) | | |
|---|---|---|
| Verbindung Nr. | Konz. mg Wirkstoff/Gefäß | Wuchshöhenm rel. |
| unbehandelt | - | 100 |
| CCC | 6 | 83,3 |
| 3 | 6 | 66,4 |

Tabelle 1b

| Sommergerste, Sorte "Aramir", Vorauflauf-Bodenbehandlung | | |
|---|---|---|
| Verbindung Nr. | Konz. mg Wirkstoff/Gefäß | Wuchshöhen rel. |
| unbehandelt | - | 100 |
| CCC | 6 | 81,0 |
| 2 | 6 | 51,5 |
| 23 | 6 | 77,2 |
| 25 | 6 | 73,6 |

Tabelle 2

| Reis, Sorte "Bahia", Nachauflauf-Bodenbehandlung | | |
|---|---|---|
| Verbindung Nr. | Konz. mg Wirkstoff/Gefäß | Wuchshöhen rel. |
| unbehandelt | - | 100 |
| CCC | 1,5 | 101,8 |
| | 6 | 99,4 |
| 3 | 1,5 | 64,2 |
| | 6 | 48,5 |

Tabelle 3

| Sommergerste, Sorte "Aramir", Nachauflauf-Blattbehandlung | | |
|---|---|---|
| Verbindung Nr. | Konz. mg Wirkstoff/Gefäß | Wuchshöhen rel. |
| unbehandelt | - | 100 |
| CCC | 6 | 84,6 |
| 2 | 6 | 71,5 |
| 3 | 6 | 64,9 |
| 23 | 6 | 70,1 |
| 25 | 6 | 74,4 |
| 27 | 6 | 69,8 |
| 32 | 6 | 71,2 |
| 34 | 6 | 70,1 |
| 36 | 6 | 71,5 |

Tabelle 4

| Rasen, "Tiergarten Mischung", Nachauflauf-Blattbehandlung | | |
|---|---|---|
| Verbindung Nr. | Konz. mg Wirkstoff/Gefäß | Wuchshöhen rel. |
| unbehandelt | - | 100 |
| CCC | 1,5 | 99,6 |
| | 6 | 99,6 |
| 3 | 1,5 | 92,5 |
| | 6 | 85,3 |
| 27 | 1,5 | 93,2 |
| | 6 | 83,6 |

Tabelle 5

| Sommerweizen, Sorte "Kolibri", Nachauflauf-Blattbehandlung | | |
|---|---|---|
| Verbindung Nr. | Konz. mg Wirkstoff/Gefäß | Wuchshöhen rel. |
| unbehandelt | - | 100 |
| CCC | 6 | 79,6 |
| 2 | 6 | 67,8 |
| 3 | 6 | 72,0 |
| 23 | 6 | 69,3 |
| 25 | 6 | 67,8 |
| 34 | 6 | 75,1 |
| 36 | 6 | 61,9 |

**Patentansprüche**

**1.** Cyclohexenonderivate der allgemeinen Formel I

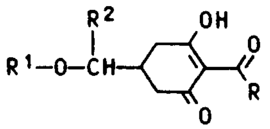

(I),

in der die Substituenten folgende Bedeutung haben:

R $C_1$-$C_6$-Alkyl, Cyclopropyl, Phenyl oder $C_2$-$C_6$-Alkoxyalkyl,

$R^1$ Wasserstoff, Tetrahydropyran-2-yl, Benzoyl oder gegebenenfalls halogensubstituiertes $C_1$-$C_6$-Alkanoyl,

$R^2$ Wasserstoff, $C_1$-$C_6$-Alkyl

und deren pflanzenphysiologisch verträglichen Salze.

**2.** Cyclohexenonderivate der allgemeinen Formel I nach Anspruch 1, in der

R $C_1$-$C_5$-Alkyl, Cyclopropyl, Phenyl oder $C_2$-$C_4$-Alkoxyalkyl,

$R^1$ Wasserstoff, Tetrahydropyran-2-yl, Benzoyl, unverzweigtes $C_1$-$C_5$-Alkanoyl oder durch Chlor ein- bis dreifach substituiertes $C_2$-$C_3$-Alkanoyl,

$R^2$ Wasserstoff oder $C_1$-$C_3$-Alkyl

und deren pflanzenphysiologisch verträglichen Salze.

**3.** Verfahren zur Herstellung der Cyclohexenonderivate der Formel I nach Anspruch 1, dadurch gekennzeichnet, daß man eine entsprechende Verbindung der Formel II

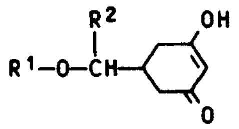 (II),

mit einem Säurechlorid R-COCl und einer Base in den Enolester überführt und diesen in Gegenwart eines sauren oder basischen Katalysators umlagert.

**4.** Verfahren zur Herstellung der Cyclohexenonderivate der Formel I nach Anspruch 1 mit der Bedeutung $R^1$ = H, dadurch gekennzeichnet, daß man eine die Schutzgruppe $R^1$ = Tetrahydropyran-2-yl, Benzoyl oder gegebenenfalls halogensubstituiertes $C_1$-$C_8$-Alkanoly enthaltende Verbindung der Formel I verseift.

**5.** Verfahren zur Herstellung eines Cyclohexenonderivates der Formel I nach Anspruch 1 mit der Bedeutung $R^1$, $R^2$ = H, dadurch gekennzeichnet, daß man einen entsprechenden Aldehyd

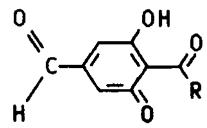

mit Natriumborhydrid umsetzt.

**6.** Mittel zur Regulierung des Pflanzenwachstums, enthaltend ein Cyclohexenonderivat der Formel I nach Anspruch 1 oder ein pflanzenphysiologisch verträgliches Salz dieser Verbindung.

**7.** Mittel zur Regulierung des Pflanzenwachstums enthaltend ein Cyclohexenonderivat der Formel I nach Anspruch 1 und inerte Zusatzstoffe.

**8.** Verfahren zur Regulierung des Pflanzenwachstums, dadurch gekennzeichnet, daß man mindestens ein Cyclohexenonderivat der Formel I nach Anspruch 1 bzw. ein Mittel nach Anspruch 6 oder 7 auf Pflanzen oder deren Lebensraum einwirken läßt.

## Claims

**1.** Cyclohexenone derivative of the formula I

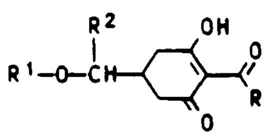 (I)

where
- R is $C_1$-$C_6$-alkyl, cyclopropyl, phenyl or $C_2$-$C_6$-alkoxyalkyl,
- $R^1$ is hydrogen, tetrahydropyran-2-yl, benzoyl or unsubstituted or halogen-substituted $C_1$-$C_6$-alkanoyl,
- $R^2$ is hydrogen or $C_1$-$C_6$-alkyl,

and phytophysiologically tolerated salts thereof.

**2.** Cyclohexenone derivative of the formula I as claimed in claim 1, where
- R is $C_1$-$C_5$-alkyl, cyclopropyl, phenyl or $C_2$-$C_4$-alkoxyalkyl,
- $R^1$ is hydrogen, tetrahydropyran-2-yl, benzoyl or straight-chain $C_1$-$C_5$-alkanoyl or is $C_2$-$C_3$-alkanoyl which is monosubstituted to trisubstituted by chlorine and
- $R^2$ is hydrogen or $C_1$-$C_3$-alkyl,

and phytophysiologically tolerated salts thereof.

3. A process for the preparation of the cyclohexenone derivative of the formula I as claimed in claim 1, wherein a corresponding compound of the formula II

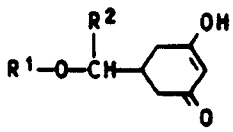

(II)

is converted with an acyl chloride R-COCl and a base into the enol ester and the latter is subjected to a rearrangement reaction in the presence of an acidic or basic catalyst.

4. A process for the preparation of a cyclohexenone derivative of the formula I as claimed in claim 1, where $R^1$ is H, wherein a compound of the formula I which contains tetrahydropyran-2-yl, benzoyl or unsubstituted or halogen-substituted $C_1$-$C_8$-alkanoyl as the protective group $R^1$ is hydrolyzed.

5. A process for the preparation of a cyclohexenone derivative of the formula I as claimed in claim 1, where $R^1$ and $R^2$ are each H, wherein a corresponding aldehyde

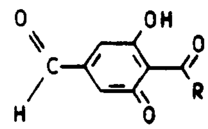

is reacted with sodium borohydride.

6. A plant growth regulator containing a cyclohexenone derivative of the formula I as claimed in claim 1 or a phytophysiologically tolerated salt of this compound.

7. A plant growth regulator containing a cyclohexenone derivative of the formula I as claimed in claim 1 and inert additives.

8. A method for regulating plant growth, wherein at least one cyclohexenone derivative of the formula I as claimed in claim 1 or an agent as claimed in claim 6 or 7 is allowed to act on plants or their habitat.

## Revendications

1. Dérivés de cyclohexénone de formule générale I

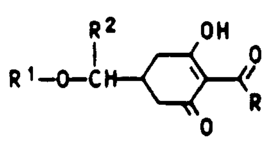

(I),

dans laquelle les substituants ont les significations suivantes :
R alkyle en C 1-C 6, cyclopropyle, phényle ou alcoxyalkyle en C 2-C 6,
$R^1$ hydrogène, tétrahydropyran-2-yle, benzoyle ou alcanoyle en C 1-C 6, éventuellement substitué par halogène,
$R^2$ hydrogène, alkyle en C 1-C 6
et leurs sels acceptables au point de vue phytophysiologique.

2. Dérivés de cyclohexénone de formule générale I, selon la revendication 1, dans laquelle
R = alkyle en C 1- C5, cyclopropyle, phényle ou alcoxyalkyle en C 2-C 4,
$R^1$ = hydrogène, tétrahydropyran-2-yle, benzoyle, alcanoyle en C 1- C 5 non ramifié ou alcanoyle en C 2-C 3, substitué une à trois fois par chlore,
$R^2$ = hydrogène ou alkyle en C 1- C 3
et leurs sels acceptables au point de vue phytophysiologique.

3. Procédé de préparation des dérivés de cyclohexénone de formule I, selon la revendication 1, caractérisé par le fait que l'on transforme un composé approprié de formule II

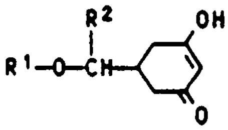

(II),

avec un chlorure d'acide R-COCl et une base en ester énolique et on transpose celui-ci en présence d'un catalyseur acide ou basique.

4. Procédé de préparation des dérivés de cyclohexénone de formule I, selon la revendication 1, avec la signification $R^1$ = H, caractérisé par le fait que l'on saponifie un composé de formule I contenant le groupe protecteur $R^1$ = tétrahydropyran-2-yle, banzoyle ou alcanoyle en C 1-C 8, éventuellement substitué par halogène.

5. Procédé de préparation d'un dérivé de cyclohexénone de formule I, selon la revendication 1, avec la signification $R^1$, $R^2$ = H, caractérisé par le fait que l'on fait réagir un aldéhyde approprié

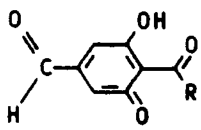

avec de l'hydrure de sodium-bore.

6. Agent pour régulariser la croissance des plantes, contenant un dérivé de cyclohexénone de formule I, selon la revendication 1, ou un sel de ce composé, acceptable au point de vue phytophysiologique.

7. Agent pour régulariser la croissance des plantes, contenant un dérivé de cyclohexénone de formule I, selon la revendication 1, ainsi que des additifs inertes.

8. Procédé pour régulariser la croissance des plantes, caractérisé par le fait que l'on fait agir sur les plantes ou leur biotope au moins un dérivé de cyclohexénone de formule I, selon la revendication 1, ou un agent selon les revendications 6 ou 7.